# EUROPEAN PATENT SPECIFICATION

(11) **EP 2 014 325 B1**
(45) Date of publication and mention of the grant of the patent: **14.04.2010**
(21) Application number: 07109746.3
(22) Date of filing: 06.06.2007
(51) Int. Cl.: A61M 15/00, A61M 16/20

(54) **One-way diaphragm valve for aerosol inhalation system for aerosol pulmonary delivery**
Einwegmembranventil für ein Aerosolinhalationssystem zur Zuführung des Aerosols zur Lunge
Soupape à diaphragme à une voie pour système d'inhalation d'aérosol pour la distribution pulmonaire d'aérosol

(43) Date of publication of application: 14.01.2009
(73) Proprietor: Air Liquide Medical Systems S.p.A., 25073 Bovezzo (IT)
(72) Inventor: Giaretta, Mariano, Liscate, MI (IT)
(74) Representative: Pittis, Olivier

(56) References cited:
- EP-A- 0 094 813
- WO-A-97/09552
- WO-A-03/097142
- US-A- 5 373 867

## Description

The present invention relates to a one-way valve that can be arranged into a medication inhaler, in particular an intended to be used in combination with both reusable and disposable hand-held single- and multi-dose aerosol drug delivery devices (ADDD) intended to deliver a metered or pre-metered aerosolized medicinal product to or by means of the human respiratory system.

One common example of an aerosol drug delivery device (ADDD) is the well-known metered dose inhaler (MDI).

These devices use a medication canister and pressurized gas to disperse a medication or a drug to be inhaled by a patient as micro-particles or -droplets that are subsequently delivered in the form of an inhalable aerosol to a patient.

These ADDD devices are usually used in combination with medication inhalers, see e.g. WO 03/097142.

A known medication inhaler is disclosed in document EP-A-0514085. It comprises an elongated aerosol holding chamber, an elastomeric diaphragm at the entering end of the elongated aerosol holding chamber to receive the L-shaped fitting carrying the medication canister, a diaphragm valve, adjacent to a mouthpiece or to a mask, adapted to open as the user inhales so as to allow the passage of medication from the holding chamber to the user. Several breaths are often required for the administration of the required drug dosage using an ADDD in combination with such a medication inhaler.

However, it has been noted in practice that an important drawback of such medication inhaler is that it can be quite difficult for the patient to open completely the diaphragm valve while inhaling so that the passage of medication through such diaphragm valve can be reduced.

Another disadvantage of such medication inhalers is that it can be quite difficult for the user to exhale, as in order to establish proper operation of the diaphragm valve, it is necessary to substantially inhibit the flow of exhale breath.

Furthermore, still another disadvantage of such inhalers is that there can be a high adhesion of medicament to the internal walls of the holding chamber resulting in a waste of medication.

It is an objective of the present invention to overcome or substantially improve at least some, but preferably all, of the above drawbacks.

A solution according to the present invention is disclosed in claim 1.

The present invention will be explained in more details in the following illustrative description of an embodiment of a one-way valve according to the invention and of an inhalation therapy device including such a one-way valve, which is made in references to the accompanying drawings among them :
- Figure 1 represents a schematic detailed view of a one-way valve according to the present invention,
- Figures 2a and 2b are schemes explaining the way the one-way valve of Figure 1 works, i.e. flaps in their rest position (Fig. 2a) and in their active position (Fig. 2b),
- Figures 3, 5, 7a and 7b show how to arrange the one-way valve of the invention on a inhalation therapy device,
- Figures 4a and 4b represents two possible embodiments of inhalation therapy devices that can incorporate the one-way valve of the invention,
- Figure 6 exhibits some details of conception of the bottom cover of an inhalation therapy devices that can incorporate the one-way valve of the invention,
- Figure 8 is a second embodiment of the valve support of Fig. 7b,
- Figure 9 shows an aerosol inhalation device incorporating the support of Figure 8,
- Figures 10a and 10b represent a cover according to the present invention, and
- Figure 11 is a view of the interior of the cover of Fig. 10a and 10b.

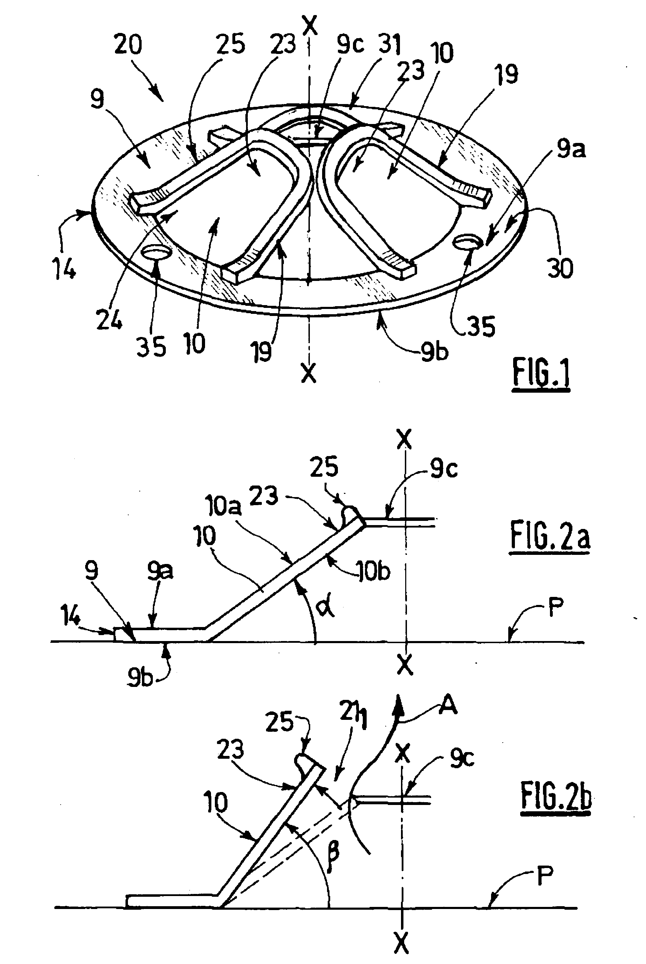

More precisely, Figure 1 is a schematic view of one embodiment of a one-way valve 20 according to the present invention that is suitable for being used in a medication inhaler, in particular an aerosol inhalation system for aerosol drug pulmonary delivery as explained below, which are also commonly called "spacers".

Said one-way valve 20 is formed by a diaphragm body 9, which is preferably thin, having an upper surface 9a, a lower surface 9b, a peripheral edge 14 and a central axis X-X in its central part 9c. In this case, the diaphragm body 9 is circular or similar.

The diaphragm body 9 further comprises here several slits 19 or cuts providing several flaps 10 in said diaphragm body 9.

The flaps 10 have an upper surface 10a, a lower surface 10b, a base 24 attached or integral to the disk-shaped diaphragm body 9 and a free mobile end 23 that is able to move from a rest position to at least an active position.

More precisely, each flap 10 can pivot and move from its rest position corresponding to the position of said flap 10 when no gas pressure is applied on its lower surface 10b, as illustrated on Figure 2a, to at least an active position corresponding to a position of said flap 10 when a gas pressure is applied on the lower surface 10b of said flap 10, as shown on Figure 2b.

In other words, as represented on Figures 2a and 2b, each flap 10 forms, with a plan P comprising the peripheral edge 14 of the diaphragm body 9 and which is perpendicular to the central axis X-X of said diaphragm body 9, a first angle α of at least about 15° when said flap 10 is in the rest position (Fig. 2a and Fig 1), and at least a second angle β greater than the first angle α (i.e. β> α) when said flap 10 is in said at least one active position, thereby defining an opening 21 allowing a passage of gas in the slit 19, i.e. between the flap 10 and the diaphragm body 9.

Indeed, as the flap 10 can pivot around its base 24, while being attached by its base 24 to the diaphragm body 9, when a gas pressure applies on its lower surface 10b (e.g. during an inhalation phase as explained below), at least the extremity 23 of said flap 10 is lifted up by the gas flow and pressure, which liberates the opening 21 and allows to the gas flow to pass through the opening 21, i.e. through the valve, from the lower surface 9b to the upper surface 9a of the diaphragm body 9 as shown by Arrow A on Figure 2b.

The value of second angle β is depending on the gas pressure applied to the lower surface 10b of the flap 10. However, the second angle β is less than 90°, preferably less than 70°.

In contrast, when no gas pressure is applied against the lower surface 10b or when a gas pressure is applied against the upper surface 10a of the flap 10 (e.g. during an exhalation phase as explained below), the flap 10 is in the rest position illustrated on Figure 2a, wherein the opening or gas passage 21 is closed by the flap 10 itself, thereby preventing any passage of gas in said gas passage or opening 21, i.e. from the upper surface 9a to the lower surface 9b of the diaphragm body 9.

Technically speaking, a flap 10 acts as a valve, whereas its seat is formed along the slit or slits 19 arranged in the diaphragm body 9.

As one can see on Figures 1 or 2, even in their rest position, the mobile extremity 23 of each flaps 10 projects on the upper surface (9a) of the diaphragm body (9), i.e. the flaps 10 are inclined (i.e. first angle α) and orientated towards the central axis XX of the diaphragm body 9.

Preferably, the diaphragm body 9 and the flaps 10 are integral and made in one piece in a flexible material, such as elastomer, rubber or silicon , e.g. by molding.

In order to improve the gas tightness, when the flaps 10 are in their rest position, and thereby preventing any passage of gas through the openings 21, i.e. between the peripheral edges of said flaps 10 and the diaphragm body 9, a reinforced lip 25 is arranged on the peripheral edge of each flaps 10. Said lip 25 increases the weight of each flap 10, which increase the gas tightness. The periphery of the flaps 10 can have a sharp or a curved shape, preferably a curved shape as shown on Figure 1.

According to a preferred embodiment, the diaphragm body 9 of the one-way valve member 20 of the present invention has a general circular shape comprising a peripheral crown-shaped part 30 delimited by the peripheral edge 9b, a central part 9c and a conical-shaped part 31 arranged between the crown-shaped part 30 and the central part 9c of the diaphragm body 9, the flaps 10 being arranged in said conical-shaped part 31.

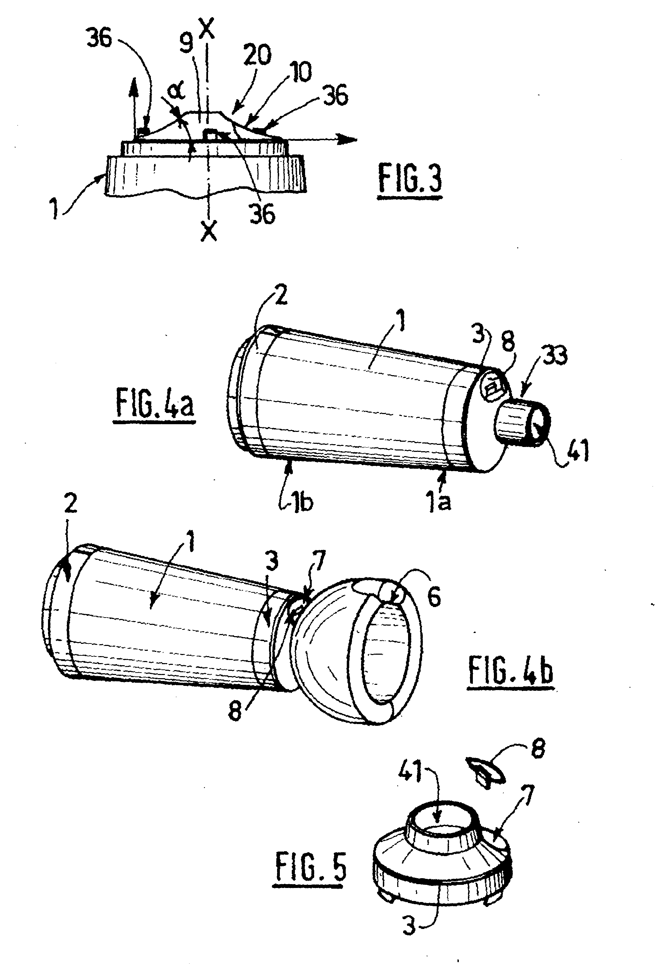

In order to easily fasten the one-way valve 20 to an inhalation therapy device, several holes 35 are provided in the crown-shaped part 30 of the diaphragm body 9, said holes 35 being able to cooperate with corresponding pins 36 or similar arranged on the main body 1 of the inhalation therapy device as illustrated on Figure 3.

Figures 4a and 4b show two inhalation therapy devices for aerosol pulmonary delivery in which the one-way valve 20 of the present invention can be mounted for controlling the flow of gases, i.e. inhaled and exhaled gases.

In both cases, the aerosol inhalation device comprises an elongated aerosol holding hollow chamber or body 1 having a first and a second end parts 2, 3 that are preferably detachable from the hollow chamber 1. The first end part is called bottom cap 2, whereas the second end part is called top part 3.

Openings are provided at both end parts 2, 3.

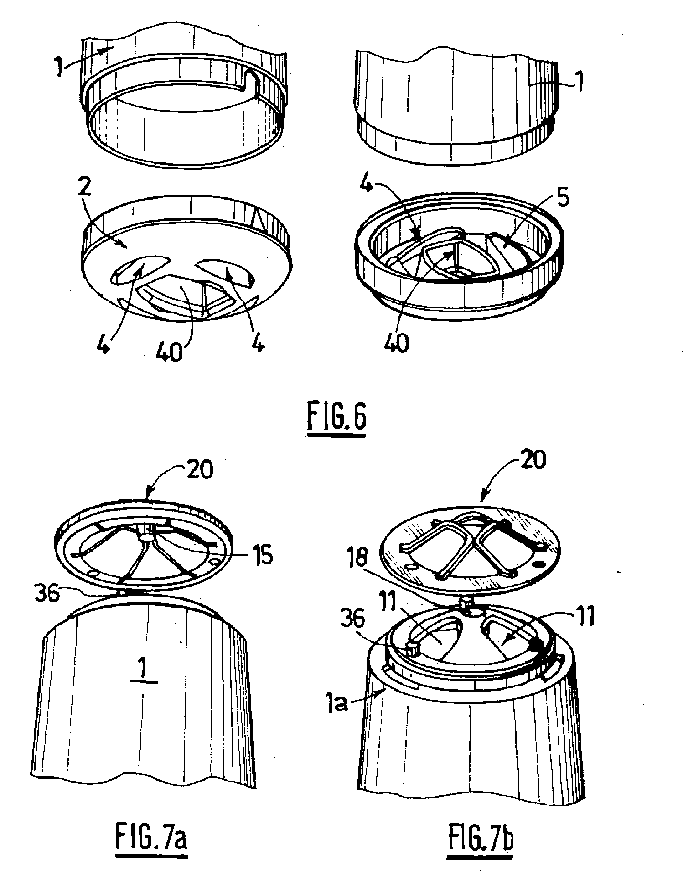

In the bottom cap 2, a central opening 40 is sized and adapted to receive a source of aerosol medicament for delivering an aerosol medication into the holding chamber 1. Besides, one or several additional air openings 4 are arranged in said bottom cap 2 around the central opening 40 for allowing passage of air into the chamber 1, while the patient is inhaling the gas and medication mixture contained into the chamber 1. Preferably, the first end part comprises at least two passageways or air openings 4, for example four openings 4, that are formed in the wall of the bottom cap 2 and elongated longitudinally in the direction of the air flow having a predetermined dimension and flexible back walls that work as a one-way closing valves 5 opened only to the inside of the housing during the inhalation phase as shown on Figure 6. In other words, the walls of the openings 4 are configured to project in the direction of the interior of the housing 1 for guiding the air flow and further said openings comprise closing valves 5 that avoid any loss of gas and medication to the atmosphere through the openings 4.

Said flexible back walls or closing valves 5 are not parallel to the plane perpendicular to the axis of said elongated aerosol holding chamber 1 in order to impart a rotational flow to the air entering the elongated aerosol holding chamber 1 through the air openings 4. Said flexible back walls 5 can also have different thicknesses one from each for the same reason. Besides, the air openings or passageways 4 can also be configured to have different shapes and sizes one from each other.

Besides, an outlet 41 is arranged into the top cap 3 for allowing the delivery of the gas and medication mixture contained into the chamber 1 to the patient's airways. This can be done thanks to a mouthpiece 33 as shown on Figure 4a or to a mask 6 as illustrated on Figure 4a.

Further, as illustrated on Figures 4a, 4b and 5, the top cap 3 also comprises a venting opening or orifice 7 in which is arranged one-way venting valve 8 that is opened only to the atmosphere during the exhalation phases, i.e. when the patient is expiring gases that are rich in CO2.

As illustrated on Figures 7a and 7b, the one-way valve 20 of the present invention is arranged in such an inhalation therapy device between the top cap 3 and the chamber 1 for regulating the gas circulation between these parts of the device.

More precisely, the conical-shaped one-way valve 20 is placed on a corresponding support 18, preferably a conical or tronco-conical support, arranged at the top end 1a of the chamber 1. Preferably, the conical support 18 projects longitudinally outside the housing 1.

Said support 18 is preferably integral to the chamber 1, e.g. formed in one piece by molding or similar.

In order to allow a gas circulation from the chamber 1 to the top cover 3, one or several gas passages 11 are arranged in the support 18 and/or between the support 18 and the internal edge of the top end 1a of the chamber 1 as shown on Figure 7b.

As already explained, pins 36 are provided which cooperate with the holes 35 of the diaphragm body 9 for maintaining said diaphragm body 9 in a fixed position in the device. The pins 36 can be arranged on the support 18.

The one-way valve 20 of the invention is opened (active position) only when gas is traveling from the chamber 1 and in the direction of the patient, due to the creation of a depression in the top cover 2 caused by the user's inhalation, thereby allowing the delivery of aerosol medicament to the patient.

In contrast, during the exhalation phases, the one-way valve 20 of the invention is closed (rest position) to block the passage of expired gases to the chamber 1, said expired gases being evacuated to the atmosphere through the one-way venting valve 8.

Preferably, the axis of said the conical support 18 and the central axis X-X are parallel to the inhalation air flow through the chamber 1. The elongated aerosol holding chamber 1 is further preferably conical, formed of a transparent material with anti-electrostatic properties

The bottom cover 2 and a top cover 3 can be threatened on the housing defining the hollow chamber 1 or fixed in any other suitable way.

The way the invention works is the following one.

First of all, the user inserts a source of an aerosol medicament or drug, such as an MDI, in the central opening 40 of the bottom cap 2 of the device of the invention and releases some aerosol medication into the holding chamber 1. As the valves 5 arranged in the openings 4 bottom cover 2 are closed, the aerosol containing the drug can not go out of the housing 1 through said openings 4.

Then, the user starts to inhale the aerosol drug by means of the mouthpiece 33 or mask 6. The user's inspiration involves a depression in the device and air will be sucked into the housing through the additional air openings 4 arranged in the bottom cover 2, thereby initiating an air flow into the hollow chamber and in the direction of the top cover 3 and of the mouthpiece 33 or mask 6, during all the time the patient is inhaling. When air is passing through the additional air openings 4, the valves 5 arranged in said openings 4 are open.

The air flow mixes with the drug aerosol comprised into the housing 1 and the air/aerosol gaseous mixture, which circulates in the direction of the patient, reaches the one-way diaphragm valve 20 of the present invention. Said gaseous flow and/or the depression caused by the user's inhalation, acts on the flaps 10 of the diaphragm body 9 which pivot from their rest position, where the passage 21 is closed, to an active position, in which the passage 21 is open, as above explained, and the flow of gas is allowed to circulates through the passages or openings 21, and afterwards reaches the patient's airways through the outlet 41. During that time, the venting valve 8 is closed.

Then, patient ceases to inhale and the expiration phase starts. During the expiration phase, the patient expires CO₂-rich gases that enter into the top cover 3 through the outlet 41. However, the expired gases can not reach the interior of the housing chamber 1 as they are blocked by the one-way diaphragm valve 20 of the present invention, as the flaps 10 are in their rest position in which the gas passages 21 are closed, and are vented to the atmosphere through the venting valve 8, which is open due to the overpressure created by the expired gas into the cover cap 3.

The flaps 10 are maintained in their rest position due to the pressure of the expired gas itself that acts on their upper surface 10b, and this is improved if a reinforced lip 25 is arranged along the flaps 10 edges as shown on figure 1.

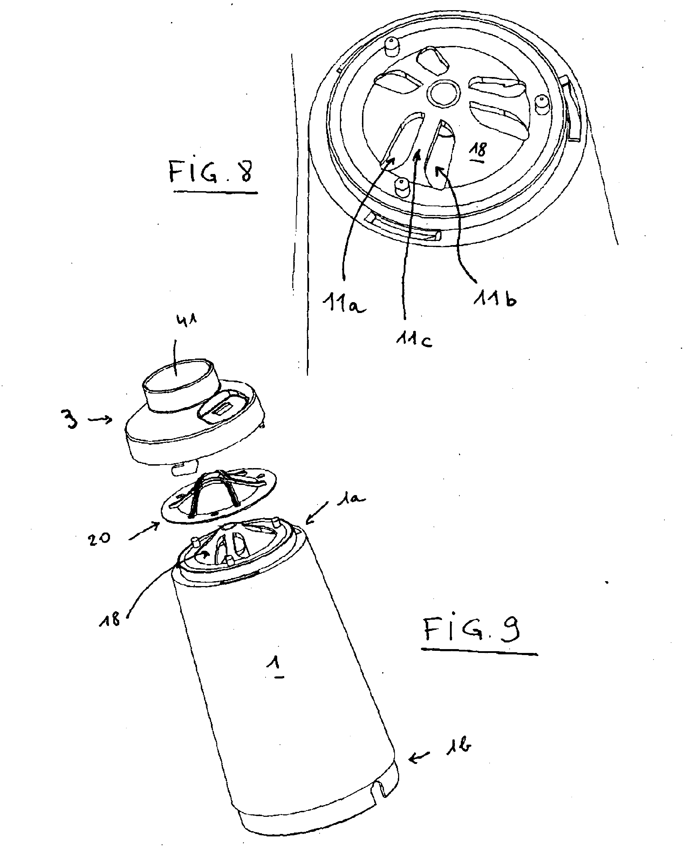

Besides, Figure 8 represents a second embodiment of the support 18 that is provided at the top end 1a of the chamber 1 for receiving the diaphragm body 9. In this embodiment, the section of the gas passages 11 arranged in the support 18 has been reduced, of for example 50%, by arranging a bridge 11c in the passage 11 thereby obtaining two sub-passages 11 a, 11b of reduced section. This embodiment can allow a better control of the gas flow and/or a better support of the flaps 10 when they are in there rest position.

Figure 9 represents a view in tri-dimension of an aerosol inhalation device for aerosol pulmonary delivery, according to the present invention, comprising a housing defining a hollow chamber 1 for receiving the aerosol and having a bottom end 1b, and a top end 1a with an outlet 41 for delivering the aerosol to the user's airways. The bottom end 1b is adapted to receive a bottom cover (as shown on Fig. 6), whereas a top cover 3 is arranged at the top end 1a. A one-way valve 20 according to the invention is arranged on the support 18 of Fig. 8, between the hollow chamber 1 and the top cover 3 as above explained.

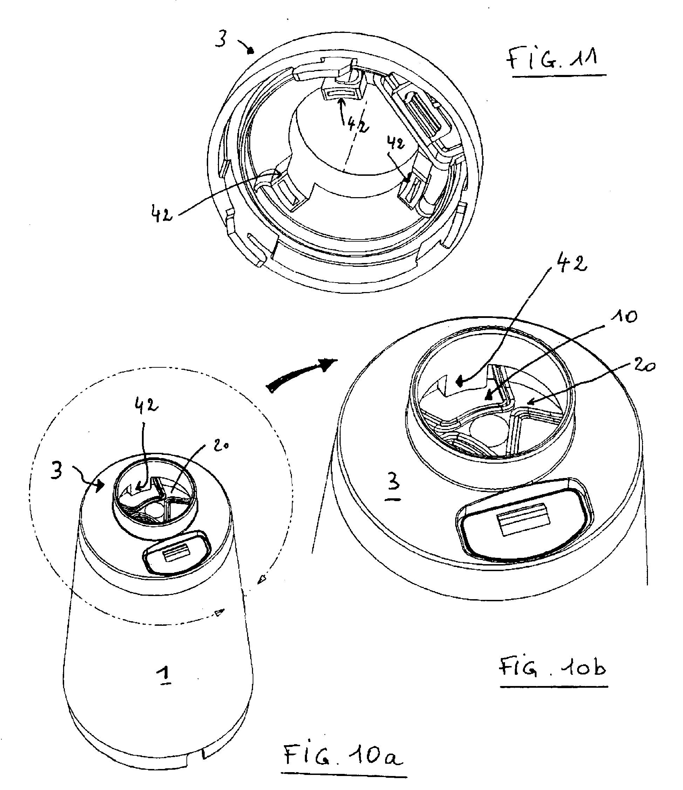

Figures 10a and 10b represent a cover 3 according to the present invention, wherein several abutments or stops 42 have been arranged in the internal wall of the cap 3.

The role of these abutments or stops 42 is to limit the opening of the valve flaps 10 when a gas flows through the valve 20, i.e. when the flaps 10 are in the active position represented on Figure 2b.

Preferably, the number of abutments or stops 42 preferably equals the number of flaps 10.

Further, the abutments or stops 42 are positioned in the cover 3 in such a way that each abutment or stop 42 is facing a flap 10 of the valve 20 thereby limiting the opening of said flap 10, when the cover 3 is fixed on the chamber 1.

As one can see on Fig. 11, the cap 3 can comprise three abutments 42 spaced of about 120°. Of course, the precise position and distance between each abutments 42 can be greater than 120° or, in contrast, less than 120°, depending on the number of abutments 42 and/or flaps 10. This can be easily determined by a skilled artisan.

The abutments 42 can be molded in one-piece with the rest of the cover 3, formed in the interior wall of the cover 3 after rough molding, or manufactured independently and inserted afterwards into it. They can also be arranged on an intermediary piece that is positioned between the cover 3 and the valve 20.

## Claims

1. Aerosol inhalation device for aerosol pulmonary delivery comprising :
- a housing defining a hollow chamber (1) for receiving an aerosol to be delivered to a user, said housing having a bottom end (1b) and a top end (1a), a bottom cover (2) being arranged at the bottom end (1b) and a top cover (3) being arranged at the top end (1a), said top cover (3) comprising an outlet (41) for delivering the aerosol to a user, and
- a one-way valve (20) arranged between the hollow chamber (1) and the top cover (3), said one-way valve (20) being formed by a diaphragm body (9) having an upper surface (9a), a lower surface (9b), a peripheral edge (14) and a central axis (XX), said diaphragm body (9) further comprising at least one slit (19) providing at least one flap (10) in said diaphragm body (9), each flap (10) having a free mobile end (23) that is able to move from a rest position to at least an active position, said at least one flap (10) forming with a plan (P) comprising the peripheral edge (14) and which is perpendicular to the central axis (XX) of the diaphragm body (9) :
- a first angle (α) of at least 15° when said at least one flap (10) is in the rest position, and
- second angle (β) greater than the first angle (α) when said at least one flap (10) is in said at least one active position,
**characterised in that** the top cover (3) further comprises one or several abutments (42) positioned in the cover (3) in such a way that each abutment (42) is facing a flap (10) of the valve (20) thereby limiting the opening of said flap (10), when the cover (3) is connected to the chamber (1).

2. Device according to claim 1, **characterized in that** the flaps (10) and the diaphragm body (9) are made in one piece.

3. Device according to any one of the proceedings claims, **characterized in that** the diaphragm body (9) is made of a flexible material, preferably rubber, elastomer or silicon.

4. Device according to any one of the proceedings claims, **characterized in that** a lip (25) is arranged on the peripheral edge of each flaps (10).

5. Device according to any one of the proceedings claims, **characterized in that** the one-way valve has a general circular shape comprising a peripheral crown-shaped part (30) delimited by a peripheral edge (9b), a central part (9c) and a conical-shaped part (31) arranged between the crown-shaped part (30) and the central part (9c) of the diaphragm body (9), the flaps (10) being arranged in said conical-shaped part (31).

6. Device according to any one of the proceedings claims, **characterized in that** the first angle (α) is of between 20 and 60° when said at least one flap (10) is in the rest position.

7. Device according to any one of the proceedings claims, **characterized in that** the first angle (α) is of less than 45° when said at least one flap (10) is in the rest position.

8. Device according to any one of the proceedings claims, **characterized in that** the second angle (β) is less than 80°, when said at least one flap (10) is in said at least one active position.

9. Device according to claim 1, **characterized in that** the number of abutments (42) equals the number of flaps (10).

10. Device according to claim 1, **characterized in that** the one-way valve (20) is supported by a support (18) arranged at the top end (1a) of the chamber (1).

11. Device according to any of claims 10, **characterized in that** the one-way valve (20) is positioned on the support (18) by means of one or several holes (35) arranged in the diaphragm body (9) or the one-way valve (20) cooperating with one or several pins (36) of the support (18), respectively.

12. Device according to any of claims 1, 10 or 11, **characterized in that** the one-way valve (20) is maintained in a fixed position on the support (18) by means of the top cover (3).

13. Device according to any of claims 1 or 10 to 12, **characterized in that** the bottom cover (2) comprises:
- a central opening (40) sized and adapted to receive a source of an aerosol drug for delivering said aerosol drug into the hollow chamber (1), and
- one or several additional air openings (4) for allowing passage of air into the hollow chamber (1), when the patient is inhaling.

14. Device according to any of claims 13, **characterized in that** the one or several additional air openings (4) of the bottom cover (2) comprises valves (5).

15. Device according to any of claims 1 or 10 to 14, **characterized in that** a mouthpiece (33) or a mask (6) is in fluid connection with the outlet (41) of the top cover (3).

16. Device according to any of claims 1 or 10 to 15, **characterized in that** the top cap (3) further comprises a venting orifice (7) comprising a one-way venting valve (8).

17. Device according to any of claims 1 or 10 to 16, **characterized in that** the housing has a cylindrical, conical or tronconical external shape.

## Patentansprüche

1. Aerosolinhalationsvorrichtung zur pulmonalen Zuführung, umfassend:
- ein Gehäuse, das eine hohle Kammer (1) zur Aufnahme eines einem Benutzer zuzuführenden Aerosols definiert, wobei das Gehäuse ein unteres Ende (1b) und ein oberes Ende (1a) aufweist, wobei eine untere Abdeckung (2) am unteren Ende (1b) und eine obere Abdeckung (3) am oberen Ende (1a) angeordnet ist, wobei die obere Abdeckung (3) einen Auslass (41) zur Zuführung des Aerosols zu einem Benutzer umfasst, und
- ein Einwegeventil (20), das zwischen der hohlen Kammer (1) und der oberen Abdeckung (3) angeordnet ist, wobei das Einwegeventil (20) durch einen Membrankörper (9) mit einer Oberseite (9a), einer Unterseite (9b), einem Umfangsrand (14) und einer mittleren Achse (XX) gebildet wird, wobei der Membrankörper (9) weiterhin mindestens einen Schlitz (19) umfasst, der mindestens eine Klappe (10) in dem Membrankörper (9) umfasst, wobei jede Klappe (10) ein freies beweglichen Ende (23) aufweist, das sich aus einer Ruhestellung in mindestens eine aktive Stellung bewegen kann, wobei die mindestens eine Klappe (10) mit einer Ebene (P), die den Umfangsrand (14) umfasst und die senkrecht zu der mittleren Achse (XX) des Membrankörpers (9) verläuft,
- einen ersten Winkel (α) von mindestens 15º bildet, wenn sich die mindestens eine Klappe (10) in der Ruhestellung befindet, und
- einen zweiten Winkel (β), der größer ist als der erste Winkel (α), bildet, wenn sich die mindestens eine Klappe (10) in der mindestens einen aktiven Position befindet,
**dadurch gekennzeichnet, dass** die obere Abdeckung (3) weiterhin einen oder mehrere Anschläge (42) umfasst, die so in der Abdeckung (3) angeordnet sind, dass jeder Anschlag (42) zu einer Klappe (10) des Ventils (20) weist, wodurch das Öffnen der Klappe (10) begrenzt wird, wenn die Abdeckung (3) mit der Kammer (1) verbunden ist.

2. Vorrichtung nach Anspruch 1, **dadurch gekennzeichnet, dass** die Klappen (10) und der Membrankörper (9) einstückig hergestellt sind.

3. Vorrichtung nach einem der vorhergehenden Ansprüche, **dadurch gekennzeichnet, dass** der Membrankörper (9) aus einem flexiblen Material, vorzugsweise Kautschuk, Elastomer oder Silikon, hergestellt ist.

4. Vorrichtung nach einem der vorhergehenden Ansprüche, **dadurch gekennzeichnet, dass** eine Lippe (25) an dem Umfangsrand jeder Klappe (10) angeordnet ist.

5. Vorrichtung nach einem der vorhergehenden Ansprüche, **dadurch gekennzeichnet, dass** das Einwegeventil eine allgemein kreisförmige Gestalt aufweist, die einen durch einen Umfangsrand (9b) begrenzten kronenförmigen Umfangsteil (30), einen mittleren Teil (9c) und einen zwischen dem kronenförmigen Teil (30) und dem Umfangsteil (9c) des Membrankörpers (9) angeordneten kegelförmigen Teil (31) aufweist, wobei die Klappen (10) in dem kegelförmigen Teil (31) angeordnet sind.

6. Vorrichtung nach einem der vorhergehenden Ansprüche, **dadurch gekennzeichnet, dass** der erste Winkel (α) zwischen 20 und 60º liegt, wenn sich die mindestens eine Klappe (10) in der Ruhestellung befindet.

7. Vorrichtung nach einem der vorhergehenden Ansprüche, **dadurch gekennzeichnet, dass** der erste Winkel (α) weniger als 45º beträgt, wenn sich die mindestens eine Klappe (10) in der Ruhestellung befindet.

8. Vorrichtung nach einem der vorhergehenden Ansprüche, **dadurch gekennzeichnet, dass** der zweite Winkel (β) weniger als 80º beträgt, wenn sich die mindestens eine Klappe (10) in der mindestens einen aktiven Position befindet.

9. Vorrichtung nach Anspruch 1, **dadurch gekennzeichnet, dass** die Anzahl von Anschlägen (42) der Anzahl von Klappen (10) entspricht.

10. Vorrichtung nach Anspruch 1, **dadurch gekennzeichnet, dass** das Einwegeventil (20) von einem Träger (18) gestützt wird, der am oberen Ende (1a) der Kammer (1) angeordnet ist.

11. Vorrichtung nach Anspruch 10, **dadurch gekennzeichnet, dass** das Einwegeventil (20) mittels eines oder mehrerer Löcher (35), die in dem Membrankörper (9) des Einwegeventils (20) angeordnet sind und mit einem bzw. mehreren Stiften (36) des Trägers (18) zusammenwirken, auf dem Träger (18) positioniert ist.

12. Vorrichtung nach einem der Ansprüche 1, 10 oder 11, **dadurch gekennzeichnet, dass** das Einwegeventil (20) mittels der oberen Abdeckung (3) in einer festgelegten Position auf dem Träger (18) gehalten wird.

13. Vorrichtung nach einem der Ansprüche 1 oder 10 bis 12, **dadurch gekennzeichnet, dass** die untere Abdeckung (2) Folgendes umfasst:
- eine mittlere Öffnung (40), die dazu bemessen und ausgeführt ist, eine Quelle eines Aerosol-Arzneimittels zur Zuführung des Aerosol-Arzneimittels in die hohle Kammer (1) aufzunehmen, und
- eine oder mehrere zusätzliche Luftöffnungen (4), dank derer Luft in die hohle Kammer (1) gelangen kann, wenn der Patient inhaliert.

14. Vorrichtung nach Anspruch 13, **dadurch gekennzeichnet, dass** die eine oder die mehreren zusätzlichen Luftöffnungen (4) der unteren Abdeckung (2) Ventile (5) umfassen.

15. Vorrichtung nach einem der Ansprüche 1 oder 10 bis 14, **dadurch gekennzeichnet, dass** ein Mundstück (33) oder eine Maske (6) mit dem Auslass (41) der oberen Abdeckung (3) in Strömungsverbindung steht.

16. Vorrichtung nach einem der Ansprüche 1 oder 10 bis 15, **dadurch gekennzeichnet, dass** die obere Abdeckung (3) weiterhin eine Entlüftungsöffnung (7) umfasst, die ein Einwege-Entlüftungsventil (8) umfasst.

17. Vorrichtung nach einem der Ansprüche 1 oder 10 bis 16, **dadurch gekennzeichnet, dass** das Gehäuse eine äußere Zylinder-, Kegel- oder Kegelstumpfform aufweist.

## Revendications

1. Dispositif d'inhalation d'aérosol pour la distribution pulmonaire d'un aérosol, comprenant:
- un boîtier qui définit une chambre creuse (1) destinée à recevoir un aérosol à distribuer à un utilisateur, ledit boîtier comprenant une extrémité inférieure (1b) et une extrémité supérieure (1a), un couvercle inférieur (2) étant agencé à l'extrémité inférieure (1b) et un couvercle supérieur (3) étant agencé à l'extrémité supérieure (1a), ledit couvercle supérieur (3) comportant une sortie (41) pour distribuer l'aérosol à un utilisateur; et
- une soupape à une voie (20) agencée entre la chambre creuse (1) et le couvercle supérieur (3), ladite soupape à une voie (20) étant formée par un corps de diaphragme (9) qui présente une surface supérieure (9a), une surface inférieure (9b), un bord périphérique (14) et un axe central (XX), ledit corps de diaphragme (9) comprenant en outre au moins une fente (10) formant au moins un volet (10) dans ledit corps de diaphragme (9), chaque volet (10) présentant une extrémité mobile libre (23) qui est capable de se déplacer d'une position de repos à au moins une position active, ledit au moins un volet (10) formant avec un plan (P) comprenant le bord périphérique (14) et qui est perpendiculaire à l'axe central (XX) du corps de diaphragme (9):
- un premier angle (α) d'au moins 15° lorsque ledit au moins un volet (10) se trouve dans la position de repos; et
- un deuxième angle (β) supérieur au premier angle (α) lorsque ledit au moins un volet (10) se trouve dans ladite au moins une position active,
**caractérisé en ce que** le couvercle supérieur (3) comporte en outre une ou plusieurs butée(s) (42) positionnée(s) dans le couvercle (3) de telle sorte que chaque butée (42) soit située en face d'un volet (10) de la soupape (20), limitant ainsi l'ouverture dudit volet (10) lorsque le couvercle (3) est connecté à la chambre (1).

2. Dispositif selon la revendication 1, **caractérisé en ce que** les volets (10) et le corps de diaphragme (9) sont formés d'une seule pièce.

3. Dispositif selon l'une quelconque des revendications précédentes, **caractérisé en ce que** le corps de diaphragme (9) est constitué d'une matière flexible, de préférence le caoutchouc, un élastomère ou du silicone.

4. Dispositif selon l'une quelconque des revendications précédentes, **caractérisé en ce qu'**une lèvre (25) est formée sur le bord périphérique de chaque volet (10).

5. Dispositif selon l'une quelconque des revendications précédentes, **caractérisé en ce que** la soupape à une voie présente une forme générale circulaire comprenant une partie périphérique en forme de couronne (30) qui est délimitée par un bord périphérique (9b), une partie centrale (9c) et une partie de forme conique (31) qui est agencée entre la partie en forme de couronne (30) et la partie centrale (9c) du corps de diaphragme (9), les volets (10) étant agencés dans ladite partie de forme conique (31).

6. Dispositif selon l'une quelconque des revendications précédentes, **caractérisé en ce que** le premier angle (α) est compris entre 20° et 60° lorsque ledit au moins un volet (10) se trouve dans la position de repos.

7. Dispositif selon l'une quelconque des revendications précédentes, **caractérisé en ce que** le premier angle (α) est inférieur à 45° lorsque ledit au moins un volet (10) se trouve dans la position de repos.

8. Dispositif selon l'une quelconque des revendications précédentes, **caractérisé en ce que** le deuxième angle (β) est inférieur à 80° lorsque ledit au moins un volet (10) se trouve dans ladite au moins une position active.

9. Dispositif selon la revendication 1, **caractérisé en ce que** le nombre de butées (42) est égal au nombre de volets (10).

10. Dispositif selon la revendication 1, **caractérisé en ce que** la soupape à une voie (20) est supportée par un support (18) qui est agencé à l'extrémité supérieure (1a) de la chambre (1).

11. Dispositif selon la revendication 10, **caractérisé en ce que** la soupape à une voie (20) est positionnée sur le support (18) au moyen d'un ou de plusieurs trou(s) 35 qui est (sont) formé(s) dans le corps de diaphragme (9) de la soupape à une voie (20) et qui coopère(nt) avec une ou plusieurs tige(s) (36) du support (18), respectivement.

12. Dispositif selon l'une quelconque des revendications 1, 10 ou 11, **caractérisé en ce que** la soupape à une voie (20) est maintenue dans une position fixe sur le support (18) au moyen du couvercle supérieur (3).

13. Dispositif selon l'une quelconque des revendications 1 ou 10 à 12, **caractérisé en ce que** le couvercle inférieur (2) comporte:
- une ouverture centrale (40) dimensionnée et adaptée pour recevoir une source d'un médicament en aérosol afin de distribuer ledit médicament en aérosol dans la chambre creuse (1); et
- une ou plusieurs ouverture(s) d'air supplémentaire(s) (4) destinée(s) à permettre le passage d'air dans la chambre creuse (1) lorsque le patient inhale.

14. Dispositif selon la revendication 13, **caractérisé en ce que** ladite/lesdites une ou plusieurs ouverture(s) d'air supplémentaire(s) (4) du couvercle inférieur (2) comprend (comprennent) des valves (5).

15. Dispositif selon l'une quelconque des revendications 1 ou 10 à 14, **caractérisé en ce qu'**un embout buccal (33) ou un masque (6) est en communication fluidique avec la sortie (41) du couvercle supérieur (3).

16. Dispositif selon l'une quelconque des revendications 1 ou 10 à 15, **caractérisé en ce que** le couvercle supérieur (3) comprend un outre un orifice d'évacuation (7) qui comprend une soupape d'évacuation à une voie (8).

17. Dispositif selon l'une quelconque des revendications 1 ou 10 à 16, **caractérisé en ce que** le boîtier présente une forme extérieure cylindrique, conique ou tronconique.
